(19) Europäisches Patentamt
European Patent Office
Office européen des brevets

(11) Veröffentlichungsnummer: **0 036 842**
**A1**

# (12) EUROPÄISCHE PATENTANMELDUNG

(21) Anmeldenummer: **81810110.7**

(22) Anmeldetag: **20.03.81**

(51) Int. Cl.³: **C 07 D 339/06**, C 07 D 409/04, A 01 N 43/28

(30) Priorität: **26.03.80 CH 2374/80**
**16.09.80 CH 6930/80**

(43) Veröffentlichungstag der Anmeldung: **30.09.81**
**Patentblatt 81/39**

(84) Benannte Vertragsstaaten: **CH DE FR GB IT LI**

(71) Anmelder: **CIBA-GEIGY AG, Patentabteilung Postfach, CH-4002 Basel (CH)**

(72) Erfinder: **Fäh, Hansjakob, Dr., Schlossweg, CH-4466 Ormalingen (CH)**
Erfinder: **Farooq, Saleem, Dr., Im Schalengarten 2, CH-4107 Ettingen (CH)**
Erfinder: **Grieder, Alfred, Dr., Ob den Reben 15, CH-4461 Böckten (CH)**
Erfinder: **Scheuzger, Karl, Markgräflerstrasse 71, CH-4057 Basel (CH)**

## (54) 1,3-Benzodithiol-2-N,N-dialkyliminium-Salze.

(57) Neue substituierte 1,3-Benzodithiol-2-N,N-dialkyliminium-Salze der Formel

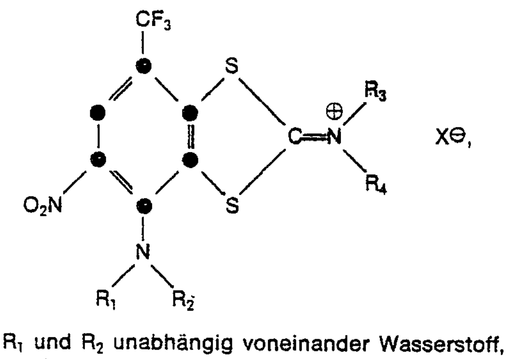

worin $R_1$ und $R_2$ unabhängig voneinander Wasserstoff, $C_1$-$C_5$-Alkyl, $C_3$-$C_6$-Cycloalkyl, $C_3$-Alkenyl, $C_3$-$C_5$-Alkinyl oder den Rest $-(CH_2)_n-OR$, wobei R für $C_1$-$C_4$-Alkyl steht und n eine Zahl 2 oder 3 ist; $R_3$ und $R_4$ unabhängig voneinander $C_1$-$C_4$-Alkyl oder zusammen den Rest $-(CH_2)_4-$ oder $-(CH_2)_5-$; und $X^\ominus$ ein Moläquivalent eines Anions einer anorganischen oder organischen Säure bedeuten; Verfahren zur Herstellung dieser Verbindungen sowie diese enthaltende Mittel zur Verwendung in der Schädlingsbekämpfung, insbesondere zur Bekämpfung von Pflanzen und Tiere befallenden Vertretern der Ordnung Akarina. Die neuen Verbindungen zeichnen sich durch spezielle Wirksamkeit gegen pflanzenbeschädigende Milben aus.

EP 0 036 842 A1

ACTORUM AG

0036842

CIBA-GEIGY AG
Basel (Schweiz)

5-12778/1-3

## 1,3-Benzodithiol-2-N,N-dialkyliminium-Salze

Die vorliegende Erfindung betrifft neue substituierte 1,3-Benzodithiol-2-N,N-dialkyliminium-Salze, Verfahren zu ihrer Herstellung und ihre Verwendung in der Schädlingsbekämpfung.

Die erfindungsgemässen neuen Iminium-Salze haben die Formel I

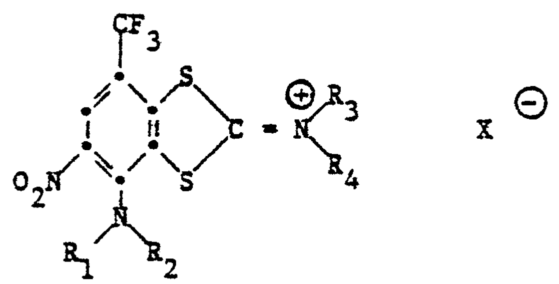

(I),

worin $R_1$ und $R_2$ unabhängig voneinander Wasserstoff, $C_1$-$C_5$-Alkyl, vorzugsweise $C_1$-$C_4$-Alkyl, $C_3$-$C_6$-Cycloalkyl, $C_3$-Alkenyl, $C_3$-$C_5$-Alkinyl oder den Rest $-(CH_2)_n-OR$, wobei R für $C_1$-$C_4$ Alkyl steht und n eine Zahl 2 oder 3 ist;
$R_3$ und $R_4$ unabhängig voneinander $C_1$-$C_4$-Alkyl, oder zusammen den Rest $-(CH_2)_4-$ oder $-(CH_2)_5-$; und
$X^{\ominus}$ ein Moläquivalent eines Anions einer anorganischen oder organischen Säure bedeuten.

Von besonderem Interesse sind erfindungsgemässe Verbindungen der Formel I, worin $R_1$ und $R_2$ unabhängig voneinander Wasserstoff, $C_1$-$C_4$-Alkyl, $C_3$-Alkenyl, $C_3$-Alkinyl oder 2-Methoxyäthyl; und $R_3$ und $R_4$ Methyl bedeuten. Wegen ihrer Wirkung als Schädlingsbekämpfungsmittel bevorzugt sind ferner Verbindungen der Formel I, die

dadurch gekennzeichnet sind, dass $R_1$ Wasserstoff bedeutet. Wertvoll sind aufgrund ihrer biologischen Wirksamkeit auch Verbindungen der Formel I, worin das Anion $X^{\ominus}$ Chlorid, Bromid, Jodid, Sulfat, Hydrogensulfat, Chlorat, Perchlorat, Rhodanid, Nitrat, Phosphat, Hydrogenphosphat, Tetrafluorborat, Acetat, Trichloracetat, Trifluoracetat, Phenylsulfonat, Oxalat, Malonat, Succinat, Malat, Tartrat oder Citrat, vorzugsweise Perchlorat, Hydrogensulfat, Jodid oder Rhodanid, bedeutet.

Aus der deutschen Offenlegungsschrift 2644036 sind bereits 1,3-Benzodithiol-2-on- und 1,3-Benzodithiol-2-thion-Verbindungen bekannt, die dort als herbizid, fungizid, akarizid, nematozid und insektizid wirksam beschrieben werden. Auf die Herstellung von 4-Nitro-6-trifluormethyl-1,3-Benzodithiol-2-N-alkyliminen wird in J. Org. Chem. 44/2, 267 ff (1979) Bezug genommen. Quaternäre Iminiumsalze von 2-Alkyliminonaphto[2,3]-1,3-dithiol-4,9-dionen und deren Herstellungsverfahren sind aus J.Am. Chem. Soc. 73, 3460 (1951) bekannt.

Ueberraschenderweise wurde nun gefunden, dass die erfindungsgemässen Verbindungen der Formel I bei guter Pflanzenverträglichkeit und geringer Warmblütertoxizität ausgezeichnete Wirksamkeit als Schädlingsbekämpfungsmittel, insbesondere als Mittel zur Bekämpfung von Pflanzen und Tiere befallenden Vertretern der Ordnung Akarina aufweisen. Die Verbindungen der Formel I eignen sich vor allem zur Bekämpfung von Vertretern der Ordnung Akarina der Familien: Ixodidae, Argasidae, Tetranychidae, Dermanyssidae, sowie auch zur Bekämpfung von Insekten der Ordnungen: Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera. Ueberraschend ist ferner, dass Verbindungen der Formel I einen ausgeprägten Blatt-Penetrations-Effekt zeigen, der sie insbesondere zur Bekämpfung von Schädlings-Spezies geeignet macht, die die Blatt-Unterseiten der befallenen Pflanzen besiedeln.

Aufgrund ihrer guten akariziden Wirksamkeit sind die Verbindungen der Formel I weiterhin zur Bekämpfung von Ektoparasiten an Haus- und Nutztieren, z.B., durch Tier-, Stall- und Weidebehandlung geeignet.

Besonders hervorzuheben ist, dass die erfindungsgemässen Verbindungen der Formel I eine überraschend hohe und spezifische Wirksamkeit gegen Pflanzen schädigende Milben und gegen tierparasitäre Milben besitzen. So können die Verbindungen der Formel I zur Bekämpfung von phytophagen Milben, z.B. der Familien Tetranychidae und Phytoptipalpidae (Spinnmilben), Tarsonemidae (Weichhautmilben) und Eriophyidae (Gallmilben), eingesetzt werden. Die Verbindungen der Formel I eignen sich vor allem zur Bekämpfung der folgenden, Obst- und Gemüsekulturen befallenden Milbenspezies: Tetranychus urticae, Tetranychus cinnabarinus, Panonychus ulmi, Bryobia rubrioculus, Panonychus citri, Eriophyes pyri, Eriophyes ribis, Eriophyes vitis, Tarsomemus pallidus, Phyllocoptes vitis und Phyllocopture oleivora. Mit Hilfe von Verbindungen der Formel I können auch tierparasitäre Milben, z.B. der Familien Sarcoptidae, Psoroptidae, Dermanyssidae und Demodicidae, insbesondere Räudemilben der Spezies Sarcoptes scabiei und Notoedres cati, welche sich tief in die Epidermis der befallenden Haus- und Nutztiere bis an die Nervenenden einbohren und intensive Irritationen und Schädigungen bei den Tieren verursachen, ferner die Spezies Dermanyssus gallinae und Psoroptes ovis bekämpft werden.

Gemäss vorliegender Erfindung werden die neuen Iminiumsalze der Formel I in der Weise hergestellt, dass man ein 2-Nitrophenyl-N,N-dialkyldithiocarbamat der Formel II

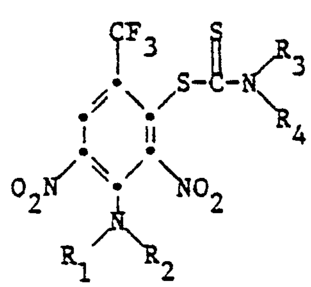

(II),

worin $R_1$, $R_2$, $R_3$ und $R_4$ die unter Formel I angegebenen Bedeutungen haben, in Gegenwart eines inerten Lösungsmittels mit einer vorzugsweise starken Säure der Formel III

$$HX \qquad (III)$$

umsetzt. Als Säure kann man z.B. Chlorwasserstoffsäure, Bromwasserstoffsäure, Jodwasserstoffsäure, Schwefelsäure, Chlorsäure, Perchlorsäure, Rhodanwasserstoffsäure, Salpetersäure, Phosphorsäure, Tetrafluorborsäure, Trichloressigsäure oder Trifluoressigsäure verwenden. Als starke Säuren werden Perchlorsäure, Schwefelsäure, Jodwasserstoffsäure und Rhodanwasserstoffsäure bevorzugt. Die Säuren können als solche oder in Form ihrer wässrigen Lösungen im Konzentrationsbereich von 30-100 % eingesetzt werden. Die Verwendung von wässrigen Lösungen der Säuren bietet insbesondere im Zusammenhang mit der Abtrennung der Iminiumsalze der Formel I von Nebenprodukten und Verunreinigungen Vorteile und ist daher bevorzugt. Die Säuren werden in mindestens stöchiometrischer Menge, vorzugsweise jedoch in einem Ueberschuss von bis zu 10 Mol pro Mol 2-Nitrophenyl-N,N-dialkyl-dithiocarbamat der Formel II,eingesetzt. Das erfindungsgemässe Verfahren wird in der Regel bei Temperaturen von 0-200°C, vorzugsweise bei 20-100°C, durchgeführt. Geeignete Lösungsmittel sind vor allem Dialkylketone mit je 1 bis 4 Kohlenstoffatomen in den Alkylgruppen und Alkylester niederaliphatischer Carbonsäuren mit 2 bis 4 Kohlenstoffatomen im Säureteil und 1 bis 4 Kohlenstoffatomen im Alkoholteil. Geeignete Dialkylketone sind insbesondere Aceton,. Methyläthylketon, Diäthylketon und Methylisobutylketon. Als geeignete Carbonsäureester sind insbesondere Aethylacetat, Butylacetat, Aethylpropionat und Methylbutyrat zu erwähnen.

Mit Hilfe von Anionenaustauschreaktionen lässt sich erwüschtenfalls bei einem erhaltenen Iminiumsalz der Formel I das vorhandene Säureanion $X^{\ominus}$ durch ein anderes, erwünschtes Säureanion, wobei es sich um das Anion einer schwachen, z.B. einer schwachen organischen Säure, handeln kann, ersetzen. Hierzu kann das zunächst erhaltene Iminiumsalz der Formel I, worin das Anion z.B. Hydrogensulfat oder Chlorid ist, mit einem Salz der dem erwünschten Anion entsprechenden Säure, z.B. einem Alkalirhodanid, in an sich bekannter Weise umgesetzt werden. Bei diesen Salzen handelt es sich bevorzugt um die entsprechenden Alkali- oder Erdalkalisalze. Die Salze werden für die Anionenaustauschreaktion im allgemeinen in stöchiometrischem Ueberschuss verwendet.

Die nach Abtrennung der Lösung des Iminiumsalzes der Formel I gelöste salpetrige Säure kann auf übliche Weise durch bekannte Reaktionen, wie Umsetzung mit Amidosulfonsäure, Harnstoff oder Hydroxylamin, durch Reaktion mit dem Lösungsmittel (Bildung von Isonitrosoverbindungen) oder durch Reduktion, beispielsweise mit schwefliger Säure, beseitigt werden. Die Beseitigung der bei der Bildung der Iminiumsalze der Formel I entstehenden salpetrigen Säure kann auch in vorteilhafter Weise in situ erfolgen, indem man die Bildung der Iminiumsalze der Formel I in Gegenwart einer Substanz, die mit salpetriger Säure reagieren kann, wie Amidosulfonsäure, Harnstoff oder Hydroxylamin, oder in Gegenwart eines Reduktionsmittels, wie schwefelige Säure, vornimmt.

Gemäss einer vorteilhaften Ausführungsform des erfindungsgemässen Verfahrens wird das 2-Nitrophenyl-N,N-dialkyldithiocarbamat der Formel II in einem aus Wasser und einem mit Wasser nicht mischbaren organischen Lösungsmittel bestehenden Reaktionsmedium in Gegenwart einer Säure der Formel III, vorzugsweise bei einer Temperatur von 0-100°C, in das entsprechende Iminiumsalz der Formel I überführt, die organische Phase abgetrennt und das in wässriger Lösung vorliegende Iminiumsalze nach Zugabe von frischem organischem Lösungsmittel und Neutralisation der überschüssigen Säure in kristalliner Form gewonnen.

Die als Ausgangsmaterialien benötigten 2-Nitrophenyl-N,N-dialkyldithiocarbamate der Formel II können durch Umsetzung der entsprechenden 2-Nitrochlorphenole mit Natrium-N,N-dialkyldithiocarbamat in bekannter Weise hergestellt werden [vgl. J. Org. Chem. 44 (2), 272 (1979)].

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen.

Als Zusätze kommen z.B. folgende Wirkstoffe in Betracht:

organische Phosphorverbindungen,
Nitrophenole und Derivate,
Formamidine, Harnstoffe, Carbamate und
chlorierte Kohlenwasserstoffe.

Mit besonderem Vorteil kann man die Verbindungen der Formel I auch mit Substanzen kombinieren, welche einen pestizid verstärkenden Effekt ausüben. Beispiele solcher Verbindungen sind u.a.: Piperonylbutoxid, Propinyläther, Propinyloxime, Propinylcarbamate und Propinylphosphonate, 2-(3,4-Methylendioxyphenoxy)-3,6,9-trioxaundecan oder S,S,S-Tributylphosphorotrithiote.

Die Verbindungen der Formel I können für sich allein oder zusammen mit geeigneten Trägern und/oder Zuschlagstoffen eingesetzt werden. Geeignete Träger und Zuschlagstoffe können fest oder flüssig sein und entsprechen den in der Formulierungstechnik üblichen Stoffen, wie z.B. natürlichen oder regenerierten Stoffen, Lösungs-, Dispergier-, netz-, Haft-, Verdickungs-, Binde- und/oder Düngemitteln. Zur Applikation können die Verbindungen der Formel I zu Stäubemitteln, Emulsionskonzentraten, Granulaten, Dispersionen, Sprays, zu Lösungen oder Aufschlämmungen in üblicher Formulierung, die in der Applikationstechnik zum Allgemeinwissen gehören, verarbeitet werden. Ferner sind "cattle dips", d.h. Viehbäder, und "spray races", d.h. Sprühgänge, in denen wässrige Zubereitungen verwendet werden zu erwähnen. Diese

Zubereitungsformen sind insbesondere zur Bekämpfung tierparasitärer Schädlinge geeignet.

Die Herstellung erfindungsgemässer Mittel erfolgt in an sich bekannter Weise durch inniges Vermischen und/oder Vermahlen von Wirkstoffen der Formel I mit den geeigneten Trägerstoffen, gegebenenfalls unter Zusatz von gegenüber den Wirkstoffen inerten Dispergier- und Lösungsmitteln. Die Wirkstoffe können in den folgenden Aufarbeitungsformen vorliegen und angewendet werden.

Feste Aufarbeitungsformen: Stäubemittel, Streumittel, Granulate (Umhüllungsgranulate, Imprägnierungsgranulate und Homogengranulate);

Flüssige Aufarbeitungsformen:

a) in Wasser dispergierbare Wirkstoffkonzentrate: Spritzpulver (wettable powders), Pasten, Emulsionen;
b) Lösungen.

Der Gehalt an Wirkstoff in den oben beschriebenen Mitteln liegt im allgemeinen zwischen 0,1 bis 95 Gew.-%.

Die Wirkstoffe der Formel I können beispielsweise wie folgt formuliert werden:

<u>Stäubemittel</u>: Zur Herstellung eines a) 5 %igen und b) 2 %igen Stäubemittels werden die folgenden Stoffe verwendet:

a) 5 Teile Wirkstoff,
95 Teile Talkum;

b) 2 Teile Wirkstoff,
1 Teil hochdisperse Kieselsäure,
97 Teile Talkum.

Die Wirkstoffe werden mit den Trägerstoffen vermischt und vermahlen.

Granulat: Zur Herstellung eines 5 %igen Granulates werden die folgenden Stoffe verwendet:

5,00 Teile Wirkstoff,
0,25 Teile epoxydiertes Pflanzenöl,
0,25 Teile Cetylpolyglykoläther,
3,50 Teile Polyäthylenglykol,
91,00 Teile Kaolin (Korngrösse 0,3-0,8 mm).

Die Aktivsubstanz wird mit dem epoxydierten Pflanzenöl vermischt und in 6 Teilen Aceton gelöst, hierauf werden Polyäthylenglykol und Cetylpolyglykoläther zugesetzt. Die so erhaltene Lösung wird auf Kaolin aufgesprüht und anschliessend das Aceton im Vakuum verdampft.

Spritzpulver: Zur Herstellung eines a) 40 %igen, b) und c) 25 %igen, d) 10 %igen Spritzpulvers werden folgende Bestandteile verwendet:

a) 40 Teile Wirkstoff,
5 Teile Ligninsulfonsäure-Natriumsalz,
1 Teil Dibutylnaphthalinsulfonsäure-Natriumsalz,
54 Teile Kieselsäure;

b) 25,0 Teile Wirkstoff,
4,5 Teile Calcium-Ligninsulfonat,
1,9 Teile Champagne-Kreide/Hydroxyäthylcellulose-Gemisch (1:1),
1,5 Teile Natrium-dibutyl-naphthalinsulfonat,
19,5 Teile Kieselsäure,
19,5 Teile Champagne-Kreide,
28,1 Teile Kaolin;

c) 25,0 Teile Wirkstoff,
2,5 Teile Isooctylphenoxy-polyoxyäthylen-äthanol,
1,7 Teile Champagne-Kreide/Hydroxyäthylcellulose-Gemisch (1:1),
8,3 Teile Natrimaluminiumsilikat,
16,5 Teile Kieselgur,
46,0 Teile Kaolin;

d) 10 Teile Wirkstoff,
3 Teile Gemisch der Natriumsalze von gesättigten Fettalkoholsulfaten,
5 Teile Naphthalinsulfonsäure/Formaldehyd-Kondensat,
82 Teile Kaolin.

Die Wirkstoffe werden in geeigneten Mischern mit den Zuschlagstoffen innig vermischt und auf entsprechenden Mühlen und Walzen vermahlen. Man erhält Spritzpulver, die sich mit Wasser zu Suspensionen jeder gewünschten Konzentration verdünnen lassen.

Emulgierbare Konzentrate: Zur Herstellung eines a) 10 %igen, b) 25 %igen und c) 50 %igen emulgierbaren Konzentrates werden folgende Stoffe verwendet:

a) 10,0 Teile Wirkstoff,
3,4 Teile epoxydiertes Pflanzenöl,
3,4 Teile eines Kombinationsemulgators, bestehend aus Fettalkoholpolyglykoläther und Alkylarylkyl-sulfonat-Calcium-Salz,
40,0 Teile Dimethylformamid,
43,2 Teile Xylol;

b) 25,0 Teile Wirkstoff,
2,5 Teile epoxydiertes Pflanzenöl,
10,0 Teile eines Alkylarylsulfonat/Fettalkohol-polyglykoläther-Gemisches,

5,0 Teile Dimethylformamid,
57,5 Teile Xylol;

c) 50,0 Teile Wirkstoff,
4,2 Teile Tributylphenol-Polyglykoläther,
5,8 Teile Calcium-Dodecylbenzolsulfonat,
20,0 Teile Cyclohexanon
20,0 Teile Xylol.

Aus solchen Konzentrationen können durch Verdünnen mit Wasser Emulsionen jeder gewünschten Konzentration hergestellt werden.

Sprühmittel: Zur Herstellung eines a) 5 %igen und b) 95 %igen Sprühmittels werden die folgenden Bestandteile verwendet:

a) 5 Teile Wirkstoff,
1 Teil epoxydiertes Pflanzenöl,
94 Teile Benzin (Siedegrenzen 160-190°C);

b) 95 Teile Wirkstoff,
5 Teile epoxydiertes Pflanzenöl.

Beispiel 1: Herstellung von 4-n-Propylamino-5-nitro-7-trifluormethyl-1,3-benzodithiol-2-N,N-dimethyliminiumhydrogensulfat.

In einem mit Rührer, Thermometer und Kühler versehenen Reaktionskolben wird zu einer Mischung von 206 g (0,5 Mol) 2,4-Dinitro-3-n-propylamino-6-trifluormethylphenyl-N,N-dimethyldithiocarbamat und 450 g Methylisobutylketon innerhalb von 30 Minuten bei 20-30°C 350 g 70 %ige Schwefelsäure (2,5 Mol) zudosiert. Anschliessend wird das Reaktionsgemisch innerhalb 1 Stunde auf 60-65°C aufgeheizt und dann 4 Stunden bei 60-65°C gerührt. Nach dem Abkühlen auf Raumtemperatur und Zugabe von 90 ml Wasser wird die obere organische Phase abgetrennt. Zur wässrigen Phase werden unter Rühren bei 0-5°C während 1 Stunde 160 g 50 %ige Natronlauge (2,0 Mol) zugefügt. Die abgeschiedenen Kristalle werden abfiltriert, mit wenig kaltem Wasser gewaschen

und im Vakuumtrockenschrank bei 60°C getrocknet. Es wird die Titel-Verbindung vom Schmelzpunkt 180-182°C erhalten.

Beispiel 2: Herstellung von 4-n-Propylamino-5-nitro-7-trifluormethyl-1,3-benzodithiol-2-N,N-dimethyliminiumperchlorat.

In einem mit Rührer, Thermometer und Rückflusskühler versehenen Reaktionskolben werden 20,6 g (0,05 Mol) 2,4-Dinitro-3-n-propylamino-6-trifluormethyl-N,N-dimethyldithiocarbamat und 2,0 g Methylisobutylketon vorgelegt und innerhalb von 30 Minuten 33 g (0,23 Mol) 70 %ige Perchloräsure bei 60-65°C zudosiert. Dann wird das Reaktionsgemisch abgekühlt und bei 0-5°C zunächst mit 20 g Wasser und dann mit 15,1 g (0,18 Mol) 50 %iger Natronlauge versetzt. Die abgeschiedenen Kristalle werden abfiltriert, mit wenig kaltem Wasser gewaschen und im Vakuumtrockenschrank bei 40°C getrocknet. Es wird die Titel-Verbindung vom Schmelzpunkt 153-156°C erhalten.

Beispiel 3: Herstellung von 4-n-Propylamino-5-nitro-7-trifluormethyl-1,3-benzodithiol-2-N,N-dimethyliminiumjodid.

11,6 g (0,025 Mol) 4-n-Propylamino-5-nitro-7-trifluormethyl-1,3-benzodithiol-2-N,N-dimethyliminiumhydrogensulfat werden in ca. 30 ml Wasser gelöst und 3,9 g (0,026 Mol) Natriumjodid in 10 ml Wasser zugegeben. Das ausgefallene Produkt wird abfiltriert und im Vakuumtrockenschrank getrocknet. Man erhält die Titel-Verbindung mit einem Schemlzpunkt von 188°-190°C.

Beispiel 4: Herstellung von 4-n-Propylamino-5-Nitro-7-trifluormethyl-1,3-benzodithiol-2-N,N-dimethyliminiumrhodanid.

4,63 g (0,01 Mol) 4-n-Propylamino-5-nitro-7-trifluormethyl-1,3-benzodithiol-2-N,N-dimethyliminiumhydrogensulfat, 25 ml Isobutylmethylketon und 10 ml Wasser werden bei Raumtemperatur vorgelegt. 2,5 g (0,025 Mol) Kaliumrhodanid, gelöst in 5 ml Wasser, werden rasch

zugegeben und das Rekationsgemisch wird während 15 Minuten bei Raumtemperatur gerührt. Das Reaktionsgemisch wird dann mit Methylenchlorid extrahiert und die abgetrennte organische Phase zweimal mit gesättigter Kochsalzlösung gewaschen, über Natriumsulfat getrocknet und bei vermindertem Druck eingeengt. Das Rohprodukt wird aus Aceton/Wasser umkristallisiert. Man erhält die Titel-Verbindung mit einem Schmelzpunkt von 132° - 133°C.

In analoger Weise wie in den vorstehenden Beispielen beschrieben werden die folgenden Verbindungen der Formel I hergestellt:

| $R_1$ | $R_2$ | $R_3$ | $R_4$ | $X^{\ominus}$ | Schmelzpunkt [°C] |
|---|---|---|---|---|---|
| $-CH_2-CH_2-OCH_3$ | H | $-CH_3$ | $-CH_3$ | $J^{\ominus}$ | 173 - 175 |
| 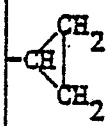 | H | $-CH_3$ | $-CH_3$ | $J^{\ominus}$ | 170 - 172 |
| $-CH_2-CH=CH_2$ | $-CH_2-CH=CH_2$ | $-CH_3$ | $-CH_3$ | $J^{\ominus}$ | 144 - 146 |
| $-CH_2-CH_2-OCH_3$ | H | $-CH_3$ | $-CH_3$ | $SCN^{\ominus}$ | 119 - 121 |
| 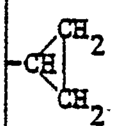 | H | $-CH_3$ | $-CH_3$ | $SCN^{\ominus}$ | 104 - 107 |
| $-CH_2-CH=CH_2$ | $-CH_2-CH=CH_2$ | $-CH_3$ | $-CH_3$ | $SCN^{\ominus}$ | 104 - 106 |
| $-CH_2-CH_2-CH_3$ | $-CH_2-CH_2-CH_3$ | $-CH_3$ | $-CH_3$ | $SCN^{\ominus}$ | 172 - 174 |
| $-CH_3$ | H | $-CH_3$ | $-CH_3$ | $J^{\ominus}$ | 205-207 |
| $-CH_3$ | H | $-CH_3$ | $-CH_3$ | $SCN^{\ominus}$ | 155-158 |
| $-C_2H_5$ | H | $-CH_3$ | $-CH_3$ | $J^{\ominus}$ | 203-205 |
| $-C_2H_5$ | H | $-CH_3$ | $-CH_3$ | $SCN^{\ominus}$ | 186-189 |
| 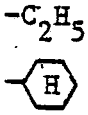 | H | $-CH_3$ | $-CH_3$ | $J^{\ominus}$ | 202-204 |
| 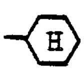 | H | $-CH_3$ | $-CH_3$ | $SCN^{\ominus}$ | 171-173 |
| $-(CH_2)_4-CH_3$ | H | $-CH_3$ | $-CH_3$ | $J^{\ominus}$ | 192-194 |
| $-(CH_2)_4-CH_3$ | H | $-CH_3$ | $-CH_3$ | $SCN^{\ominus}$ | 119-121 |

Beispiel 5: Wirkung gegen pflanzenschädigende Akariden Tetranychus urticae (OP-sensible) und Tetranychus cinnabarinus (OP-tolerant).

Die Primärblätter von Phaseolus vulgaris-Pflanzen wurden 16 Stunden vor dem Versuch auf akarizide Wirkung mit einem infestierten Blattstück aus einer Massenzucht von Tetranychus urticae (OP-sens.) oder Tetranychus cinnabarinus (OP-tol.) belegt. (Die Toleranz bezieht sich auf die Verträglichkeit gegenüber Diazinon).

Die so behandelten infestierten Pflanzen wurden mit einer Versuchslösung enthaltend 400, 200 oder 12,5 ppm der zu prüfenden Verbindung bis zur Tropfnässe besprüht.

Nach 24 Stunden und wiederum nach 7 Tagen wurden Imagines und Larven (alle beweglichen Stadien) unter dem Binokular auf lebende und tote Individuen ausgewertet.

Man verwendete pro Konzentration und pro Testspezies eine Pflanze. Während des Versuchsverlaufs standen die Pflanzen in Gewächshauskabinen bei 25°C.

Verbindungen gemäss den Beispielen 1 bis 4 zeigen in diesem Versuch gute Wirkung gegen Tetranychus urticae und Tetranychus cinnabarinus. Die Verbindung gemäss Beispiel 4 zeigte 80-100% Wirksamkeit bei 12,5 ppm gegen die geprüften Schädlingsspezies.

Beispiel 6: Pflanzenmitizide Blattpenetrations-Wirkung auf Tetranychus cinnabarinus und Panonychus citri.

Es wurden für den Versuch eingetopfte Buschbohnenpflanzen, die durch Tetranychus cinnabarinus befallen waren, und eingetopfte Citruspflanzen, die durch Panonychus citri befallen waren, verwendet.

Die Blattoberseiten der mit diesen Milben infestierten Versuchspflanzen wurden mit einer Emulsionszubereitung enthaltend 800 ppm des zu prüfenden Wirkstoffes besprüht. Nach dem Antrocknen des Sprühbelages wurde der Rand der Blattoberseite einer Anzahl befallener Blätter mit einem Wulst aus Leim (Pomona-Raupenleim) abgegrenzt, um ein Ueberlaufen der Milben von der Blattunterseite auf die Blattoberseite zu verhindern.

Die behandelten Pflanzen wurden dann im Gewächshaus bei einer Temperatur von 25° bis 29°C gehalten. Fünf Tage nach der Wirkstoffapplikation wurde festgestellt, ob eine translaminare Wirkung, d.h. Blattpenetration des Wirkstoffs, eingetreten war, und zwar durch Bestimmung der Mortalität der postembryonalen und adulten Stadien.

Verbindungen gemäss den vorstehenden Beispielen 1 bis 4 zeigten gute Wirkung in obigem Test. Die Verbindung gemäss Beispiel 4 erwies sich als 80-100% wirksam gegen die geprüften Schädlingsspezies.

Beispiel 7: Wirkung gegen tierparasitäre Milben

Von mit Dermanyssus gallinae befallenen Hühnern wurden Ansätze bestehend aus etwa 50 Milben verschiedener Stadien (Larven, Nymphen und Imagines) entnommen. Die Milbenansätze wurden jeweils in einer Verdünnungsreihe mit einer wässrigen Emulsion, Suspension bzw. Lösung des zu prüfenden Wirkstoffes benetzt. Hierzu wurden die Milbenansätze in einem Teströhrchen mit der den Wirkstoff enthaltenden flüssigen Zubereitung übergossen; die Flüssigkeit wurde anschliessend mit Hilfe eines Wattebausches aufgesogen. Die so behandelten Milben verblieben während 72 Stunden in dem Teströhrchen. Nach dieser Zeit wurde zur Auswertung die minimale, für eine 100 %ige Abtötung der behandelten Milben erforderliche Wirkstoffkonzentration gegenüber unbehandelten Kontrollansätzen ermittelt.

Verbindungen der Formel I gemäss den vorstehenden Beispielen 1 bis 4 zeigten gute Wirkung im obigen Test.

Beispiel 8: Wirkung gegen Zecken

A) Rhipicephalus bursa

Je 5 adulte Zecken oder 50 Zeckenlarven wurden in ein Glasröhrchen gezählt und für 1 bis 2 Minuten in 2 ml einer wässrigen Emulsion aus einer Verdünnungsreihe mit je 100, 10, 1 oder 0,1 ppm Testsubstanz getaucht. Das Röhrchen wurde dann mit einem genormten Wattebausch verschlossen und auf den Kopf gestellt, damit die Wirkstoffemulsion von der Watte aufgenommen werden konnte.

Die Auswertung erfolgte bei den Adulten nach 2 Wochen und bei den Larven nach 2 Tagen. Für jeden Versuch liefen 2 Wiederholungen.

B) Boophilus microplus (Larven)

In einer analogen Verdünnungsreihe wie beim Test A wurden mit je 20 sensiblen resp. OP-resistenten Larven Versuche durchgeführt. (Die Resistenz bezieht sich auf die Verträglichkeit gegenüber Diazinon).

Verbindungen der Formel I gemäss den Beispielen 1 bis 4 waren in diesen Tests gegen Adulte und Larven von Rhipicephalus bursa und sensible resp. OP-resistente Larven von Boophilus microplus wirksam.

Beispiel 9: Wirkung gegen Musca domestica

Je 50 frisch zubereitetes CSMA-Nährsubstrat für Maden wurde in Becher eingewogen. Von einer 1 Gew.-%igen acetonischen Lösung des betreffenden Wirkstoffes wurden bestimmte Mengen auf das in den Bechern befindliche Nährsubstrat pipettiert. Nach dem Durchmischen des Substrates liess man das Aceton mindestens 20 Stunden lang verdampfen.

Dann wurden pro Wirkstoff und Konzentration je 25 eintägige Maden von Musca domestica in die das so behandelte Nährsubstrat

enthaltenden Becher gegeben. Nachdem sich die Maden verpuppt hatten, wurden die gebildeten Puppen durch Ausschwemmen mit Wasser von dem Substrat abgetrennt und in mit Siebdeckeln verschlossenen Gefässen deponiert.

Die pro Ansatz ausgeschwemmten Puppen wurden gezählt (toxischer Einfluss des Wirkstoffes auf die Madenentwicklung). Dann wurde nach 10 Tagen die Anzahl der aus den Puppen geschlüpften Fliegen bestimmt. Die Auswertung erfolgte auf der Basis der noch wirksamen minimalen Konzentration der geprüften Verbindung.

Verbindungen der Formel I gemäss den Beispielen 1 bis 4 zeigten gute Wirkung im obigen Test.

Beispiel 10: Wirkung gegen Aëdes aegypti

Auf die Oberfläche von 150 ml Wasser, das sich in einem Behälter befindet, wurde so viel einer 0,1 %igen acetonischen Lösung des Wirkstoffes pipettiert, dass Konzentrationen von je 10,5 und 1 ppm erhalten wurden. Nach Verdunsten des Acetons wurde der Behälter mit 30-40 3-tägigen Aëdes-Larven beschickt. Nach 1, 2 und 5 Tagen wurde die Mortalität geprüft.

Verbindungen der Formel I gemäss den Beispielen 1 bis 4 zeigten in diesem Test gute Wirkung gegen Aëdes aegypti.

Patentansprüche

1. Verbindung der Formel I

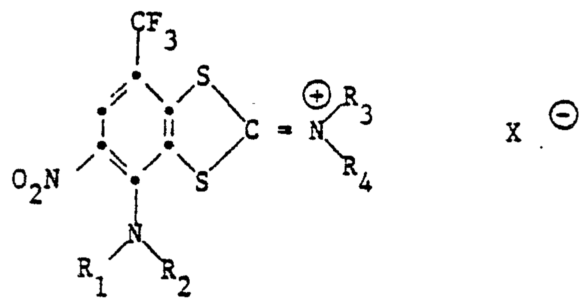

.(I),

worin $R_1$ und $R_2$ unabhängig voneinander Wasserstoff, $C_1$-$C_5$-Alkyl, $C_3$-$C_6$-Cycloalkyl, $C_3$-Alkenyl, $C_3$-$C_5$-Alkinyl oder den Rest $-(CH_2)_n-OR$, wobei R für $C_1$-$C_4$-Alkyl steht und n eine Zahl 2 oder 3 ist;

$R_3$ und $R_4$ unabhängig voneinander $C_1$-$C_4$-Alkyl oder zusammen den Rest $-(CH_2)_4-$ oder $-(CH_2)_5-$; und

$X^{\ominus}$ ein Moläquivalent eines Anions einer anorganischen oder organischen Säure bedeuten.

2. Verbindung der Formel I nach Anspruch 1, dadurch gekennzeichnet, dass $R_1$ und $R_2$ unabhängig voneinander Wasserstoff, $C_1$-$C_4$-Alkyl, $C_3$-$C_6$-Cycloalkyl, $C_3$-Alkenyl, $C_3$-$C_5$-Alkinyl oder den Rest $-(CH_2)_n-OR$, wobei R für $C_1$-$C_4$-Alkyl steht und n eine Zahl 2 oder 3 ist, bedeuten.

3. Verbindung der Formel I nach Anspruch 2, dadurch gekennzeichnet, dass $R_1$ und $R_2$ unabhängig voneinander Wasserstoff, $C_1$-$C_4$-Alkyl, $C_3$-Alkenyl, $C_3$-Alkinyl oder 2-Methoxyäthyl; und $R_3$ und $R_4$ Methyl bedeuten.

4. Verbindung der Formel I nach Anspruch 1 bis 3, dadurch gekennzeichnet, dass $R_1$ Wasserstoff bedeutet.

5. Verbindung der Formel I nach Anspruch 1 bis 4, dadurch gekennzeichnet, dass $X^{\ominus}$ Chlorid, Bromid, Jodid, Sulfat, Hydrogensulfat, Chlorat, Perchlorat, Rhodanid, Nitrat, Phosphat, Hydrogenphosphat,

Tetrafluorborat, Acetat, Chloracetat, Trichloracetat, Trifluoracetat, Phenylsulfonat, Oxalat, Malonat, Succinat, Malat, Tartrat oder Citrat bedeutet.

6. Verbindung der Formel I nach Anspruch 5, dadurch gekennzeichnet, dass $X^{\ominus}$ Perchlorat, Hydrogensulfat, Jodid oder Rhodanid bedeutet.

7. Verbindung nach Anspruch 4 der Formel Ia

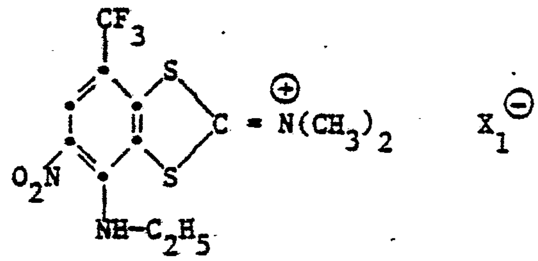

(Ia),

worin $X_1^{\ominus}$ Perchlorat, Hydrogensulfat, Jodid oder Rhodanid bedeutet.

8. Verbindung nach Anspruch 4 der Formel

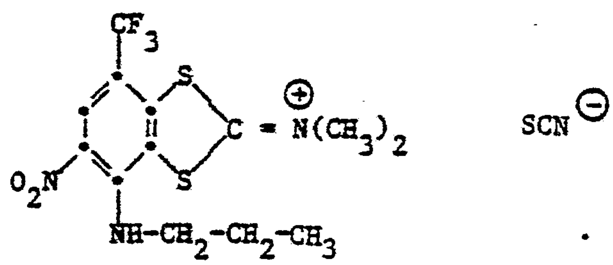

9. Verfahren zur Herstellung einer Verbindung gemäss den Ansprüchen 1 bis 8, dadurch gekennzeichnet, dass man eine Verbindung der Formel II

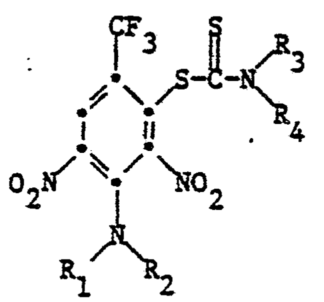

(II)

mit einer Säure der Formel III

$$HX \qquad (III)$$

umsetzt, wobei in den Formeln II und III $R_1$, $R_2$, $R_3$, $R_4$ und $X^{\ominus}$ die in den Ansprüchen 1 bis 7 angegebenen Bedeutungen haben; und dass man gegebenenfalls in der erhaltenen Verbindung der Formel I das Anion $X^{\ominus}$ mittels einer Anionenaustauschreaktion durch ein anderes Anion $X^{\ominus}$ austauscht.

10. Schädlingsbekämpfungsmittel, welches als aktive Komponente mindestens eine Verbindung gemäss den Ansprüchen 1 bis 8 zusammen mit geeigneten Trägern und/oder anderen Zuschlagstoffen enthält.

11. Verwendung von Verbindungen gemäss den Ansprüchen 1 bis 8 zur Bekämpfung von Schädlingen, vorzugsweise von Vertretern der Ordnung Akarina, insbesondere von pflanzenschädigenden Milben.

0036842

Nummer der Anmeldung

Europäisches Patentamt

# EUROPÄISCHER RECHERCHENBERICHT

EP 81 81 0110

| Kategorie | EINSCHLÄGIGE DOKUMENTE<br>Kennzeichnung des Dokuments mit Angabe, soweit erforderlich, der maßgeblichen Teile | betrifft Anspruch | KLASSIFIKATION DER ANMELDUNG (Int. Cl.³) |
|---|---|---|---|
| | US - A - 3 342 835 (T.A. LIES et al.)<br>* Spalten 1-2 *<br>-- | 1 | C 07 D 339/06<br>C 07 D 409/04<br>A 01 N 43/28 |
| | DE - A - 2 834 061 (THE ANSUL CO.)<br>* Ansprüche *<br>-- | 1,9-11 | |
| D | DE - A - 2 644 036 (THE ANSUL CO.)<br>* Ansprüche *<br>-- | 1,9-11 | RECHERCHIERTE SACHGEBIETE (Int. Cl.³) |
| D | JOURNAL OF ORGANIC CHEMISTRY, band 44, no. 2, 19-01-1979, Washington, DC,USA<br>K. RASHEED et al. "Study of the thermal decomposition of Dinitro phenyl, N,N-Dialkyldithiocarbamates and related compounds", Seiten 267-274.<br>* Seite 268 *<br>-- | 1 | C 07 D 339/06<br>C 07 D-409/04<br>A 01 N 43/28 |
| D | JOURNAL OF THE AM. CHEM. SOC., Band 43, no. 7, 06-07-1951, Am.Chem. Soc. USA<br>N.K. SUNDHOLM et al. "The reaction of 2,3-Dichloro-1,4-naphthoquinone with salts of alkyl substituted and unsubstituted dithiocarbamic acids", Seiten 3459-3463<br>* Seiten 3459-3461 *<br>-- | 1 | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument |
| P | EP - A - 0 018 578 (CIBA-GEIGY)<br>* Patentansprüche *<br>---- | 1-11 | &: Mitglied der gleichen Patentfamilie, übereinstimmendes Dokument |

Der vorliegende Recherchenbericht wurde für alle Patentansprüche erstellt.

| Recherchenort | Abschlußdatum der Recherche | Prüfer |
|---|---|---|
| Den Haag | 12.06.1981 | BRIGHENTI |

EPA form 1503.1 06.78